(19) Europäisches Patentamt
European Patent Office
Office européen des brevets

(11) **EP 4 410 179 A1**

(12) **EUROPEAN PATENT APPLICATION**
published in accordance with Art. 153(4) EPC

(43) Date of publication:
**07.08.2024  Bulletin 2024/32**

(21) Application number: **22888946.5**

(22) Date of filing: **19.08.2022**

(51) International Patent Classification (IPC):
**A61B 1/005** (2006.01)

(52) Cooperative Patent Classification (CPC):
**A61B 1/005**

(86) International application number:
**PCT/CN2022/113677**

(87) International publication number:
**WO 2023/077913 (11.05.2023 Gazette 2023/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.11.2021  CN 202111308813
08.11.2021  CN 202122726820 U**

(71) Applicant: **ZHEJIANG HEALNOC TECHNOLOGY
CO., LTD.**
**Hangzhou Zhejiang 310056 (CN)**

(72) Inventors:
• **LUO, Zhengchun**
  **Hangzhou, Zhejiang 310056 (CN)**

• **WANG, Lingang**
  **Hangzhou, Zhejiang 310056 (CN)**
• **XIAO, Gongqiang**
  **Hangzhou, Zhejiang 310056 (CN)**
• **LI, Yanhua**
  **Hangzhou, Zhejiang 310056 (CN)**
• **LI, Ke**
  **Hangzhou, Zhejiang 310056 (CN)**
• **YAO, Weizhong**
  **Hangzhou, Zhejiang 310056 (CN)**

(74) Representative: **Viering, Jentschura & Partner
mbB**
**Patent- und Rechtsanwälte**
**Am Brauhaus 8**
**01099 Dresden (DE)**

(54) **SNAKE BONE UNIT, SNAKE BONE TUBE, INSERTION CATHETER, AND ELECTRONIC
ENDOSCOPE**

(57)    A snake bone unit, a snake bone tube, an insertion catheter, and an electronic endoscope are provided. The snake bone unit includes an annular tube (100), one end of the annular tube (100) is fixed to a rotating block (21), and the other end of the annular tube (100) is provided with a rotating groove (22), and the rotating block (21) is capable of being connected with a rotating groove (22) of other snake bone units and rotating relative to the rotating groove (22) of other snake bone units; two sides of the rotating groove (22) are fixedly provided with a limiting sliding block (221), respectively, two sides of the rotating block (21) are provided with a limiting sliding groove (211), respectively, and the limiting sliding block (211) is capable of being connected to a limiting sliding groove (221) of the other snake bone units and sliding relative to the limiting sliding groove (221) of the other snake bone units; the rotating block (21) is fixedly provided with at least one auxiliary sliding block (212), the annular tube (100) is further provided with an auxiliary sliding groove (222), the number of the auxiliary sliding grooves (222) corresponds to the number of the at least one auxiliary sliding blocks (212), the auxiliary sliding groove (222) is in communication with the rotating groove (22), and the at least one auxiliary sliding blocks (212) are capable of matching with an auxiliary sliding groove of the other snake bone units and sliding relative to the auxiliary sliding groove (222) of the other snake bone units.

FIG. 1

**Description**

**CROSS-REFERENCE TO RELATED APPLICATIONS**

This application claims priority to Chinese Patent Application No. 202111308813.3, filed on November 5, 2021, entitled "INSERTION CATHETER AND ELECTRONIC ENDOSCOPE PROVIDED WITH SAME" and Chinese Patent Application No. 202122726820.7, filed on November 8, 2021, entitled "SNAKE BONE UNIT AND SNAKE BONE TUBE PROVIDED WITH SAME". The content of the above identified application is hereby incorporated herein in their entireties by reference.

**TECHNICAL FIELD**

The present invention relates to the field of endoscopes, and in particular, to a snake bone unit, a snake bone tube, an insertion catheter, and an electronic endoscope.

**BACKGROUND**

When an electronic endoscope is used, an insertion catheter is generally inserted into human body cavity and viscera cavity for direct observation, diagnosis and treatment. Therefore, most of the insertion catheters of the electronic endoscope need to have a function of bending and viewing, so as to facilitate insertion into human body and convenient observation and operation by doctors.

In the related art, a snake bone tube of the insertion catheter of the electronic endoscope generally has an instrument channel, which is configured to convey various auxiliary medical instruments for diagnosis and treatment, and inject physiological saline to dilute blood, so as to ensure that collected images are clear and tiny stones are drained out of the body.

However, space in the instrument channel will become less after corresponding instruments are installed in the instrument channel, and an injection of liquid merely through the remaining space of the instrument channel will make an injection efficiency of physiological saline lower, which may affect the doctor's operation progress because the injection efficiency of physiological saline cannot meet the operation needs.

**SUMMARY**

According to various embodiments of the present application, a snake bone unit, a snake bone tube, an insertion catheter, and an electronic endoscope are provided.

The present invention provides a snake bone unit. The snake bone unit includes an annular tube, one end of the annular tube is fixed with a rotating block, and the other end of the annular tube is provided with a rotating groove, and the rotating block is capable of being connected with a rotating groove of other snake bone units and rotating relative to the rotating groove of other snake bone units. Two sides of the rotating groove are fixedly provided with a limiting sliding block, respectively. Two sides of the rotating block are provided with a limiting sliding groove, respectively, and the limiting sliding block is capable of being connected to a limiting sliding groove of the other snake bone units and sliding relative to the limiting sliding groove of the other snake bone units. The rotating block is fixedly provided with at least one auxiliary sliding block, the annular tube is correspondingly provided with auxiliary sliding grooves with a same number as the at least one auxiliary sliding block, the auxiliary sliding groove is in communication with the rotating groove, and the auxiliary sliding block is capable of matching with an auxiliary sliding groove of the other snake bone units and sliding relative to the auxiliary sliding groove of the other snake bone units.

In a snake bone unit above, a strength of a connecting portion of the snake bone tube is increased by the auxiliary sliding groove and the auxiliary sliding block, an original overall strength of the snake bone tube can be maintained by a snake bone unit with a less thickness. The less a thickness of the snake bone unit is, the larger a cross-sectional area of the snake bone unit is, the larger an available space of the instrument channel in the snake bone tube for accommodating instrument, so as to better meet requirements of liquid injection or instrument access.

The present invention further provides a snake bone tube, a cross section of the snake bone tube is oval-shaped, and a conducting wire and at least two channels are arranged in the snake bone tube, and the conducting wire and the at least two channels penetrate through the snake bone tube along an axis of the snake bone tube.

It can be understood that compared with providing one channel, by providing at least two channels, a gap space when one channel is provided can be utilized, so that an overall cross-sectional area of the channel is larger and an utilization rate of a space in the snake bone tube is greater under a condition that an inner diameter of the snake bone tube is unchanged. In addition, a cross section of the snake bone tube is oval-shaped, so that when a plurality of channels is arranged in the snake bone tube, the gap space in the snake bone tube is less than that when a cross section is circular-shaped with the same area, and a space utilization rate in the snake bone tube is greater, so that requirements of liquid injection or instrument access can be better met.

The present invention further provides an insertion catheter. The insertion catheter includes a bending portion and a front end portion, the front end portion is provided with an image sensor and a luminous source. The bending portion includes a flexible tube and the above snake bone tube, the snake bone tube is wrapped in the flexible tube, the at least two channels penetrate through the front end portion, and the conducting wire is electrically connected with the image sensor and the luminous source.

The present invention further provides an electronic endoscope. The electronic endoscope includes an operating handle, an inserting portion and the above insertion catheters. One end of the inserting portion is connected with the bending portion, and the other end is connected with the operating handle, and the operating handle is provided with a toggling handle for controlling the bending portion to bend.

Details of one or more embodiments of this application are presented in the attached drawings and descriptions below. And other features, purposes and advantages of this application will become apparent from the description, drawings and claims.

## BRIEF DESCRIPTION OF THE DRAWINGS

For a better description and illustration of embodiments and/or examples of those inventions disclosed herein, reference may be made to one or more attached drawings. Additional details or examples used to describe the drawings should not be considered as limiting the scope of any of the disclosed inventions, currently described embodiments and/or examples, and currently understood best modes of these inventions.

FIG. 1 is a top view of a snake bone unit in the present invention.
FIG. 2 is a schematic sectional view of the snake bone unit along a direction A-A in FIG. 1.
FIG. 3 is a schematic view of a three-dimensional structure of a snake bone tube in one embodiment in the present invention.
FIG. 4 is a comparison between sectional schematic views of the snake bone unit in FIG. 2 and a snake bone unit in the related art.
FIG. 5 is a top view of the snake bone tube in FIG .3.
FIG. 6 is a stereo schematic view of the three-dimensional structure of an insertion catheter in the present invention.
FIG. 7 is a schematic view of a cross-section of a bending portion in FIG .6.
FIG. 8 is a stereo schematic view of the insertion catheter in FIG. 6 when a medical instrument is installed.
FIG. 9 is a front view of an electronic endoscope in the present invention.

## DETAILED DESCRIPTION

An electronic endoscope is widely used in urological or respiratory surgery. In urological surgery, an insertion catheter of the electronic endoscope is sterilized, entered from an urethra of a patient, passed through a bladder and a ureter, and finally entered into a renal pelvis for stone removal, examination, and biopsy. In respiratory surgery, after the insertion catheter is sterilized, the insertion catheter is inserted from an oral cavity to a trachea and a bronchus, so as to obtain a foreign body, observe a focus, and a biopsy.

In the related art, a snake bone tube of the insertion catheter of the electronic endoscope mostly have one instrument channel, which is configured for conveying various auxiliary medical devices for diagnosis and treatment, and injecting physiological saline to dilute blood, ensuring clear images and draining tiny stones out of body.

In practical use, such as ureterolithotomy or renal pelvis lithotripsy in urology, because a space of the instrument channel becomes less after instruments such as a holmium laser fiber or a stone basket are installed in the instrument channel, an injection efficiency of physiological saline is low only through a remaining space of the instrument channel, which may affect the doctor's operation.

In addition, due to a limited expansion degree of each channel in the human body, in order to avoid a situation that the insertion catheter cannot enter a corresponding natural channel in the human body, an external diameter of the insertion catheter is strictly limited and cannot be increased at will. Thus, an external diameter of the instrument channel disposed in the snake bone tube cannot be increased at will, which leads to the above problems being more difficult to solve.

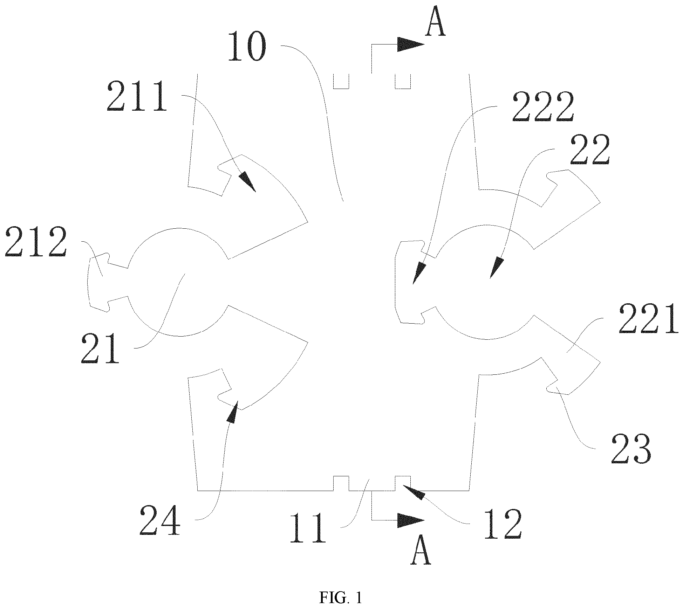

Referring to FIG. 1, in view of the above problems, the present invention firstly provides a snake bone unit which includes an annular tube 100, one end of the annular tube 100 is fixed with a rotating block 21, and the other end of the annular tube 100 is provided with a rotating groove 22, and the rotating block 21 is capable of being connected with a rotating groove 22 of other snake bone units and rotating relative to the rotating groove 22 of other snake bone units. Two sides of the rotating groove 22 are fixedly provided with one limiting sliding block 221, respectively. Two sides of the rotating block 21 are provided with one limiting sliding groove 211, respectively, and the limiting sliding block 221 is capable of being connected to a limiting sliding groove 211 of the other snake bone units and sliding relative to the limiting sliding groove 211 of the other snake bone units. The rotating block 21 is fixedly provided with at least one auxiliary sliding block 212. The annular tube 100 is further provided with an auxiliary sliding groove 222, the number of the auxiliary sliding grooves 222 corresponds to the number of the auxiliary sliding blocks 212, and the auxiliary sliding groove 222 is in communication with the rotating groove 22. The auxiliary sliding block 212 is capable of matching with an auxiliary sliding groove 222 of the other snake bone units and sliding relative to the auxiliary sliding groove 222 of the other snake bone units.

For convenience of description, in the present invention, a rotating groove 22 of one snake bone unit, an auxiliary sliding groove 222 of one snake bone unit and a limiting sliding block 221 of one snake bone unit, and a rotating block 21 of another snake bone unit corresponding to the snake bone unit, an auxiliary sliding block 212 of another snake bone unit corresponding to the snake bone unit and a limiting sliding groove 211 of another snake bone unit corresponding to the snake bone unit are defined as a connecting portion of two adjacent snake bone units.

In the present invention, the rotating block 21 of a snake bone unit is connected to and capable of rotating relative to the rotating groove 22 of another snake bone unit, so that the two adjacent snake bone units are capable of rotating relatively to each other, thus meeting a movable requirements of the snake bone tube.

Through a slidably connection between the limiting sliding block 221 of one snake bone unit and the limiting sliding groove 211 of another snake bone units, on one hand, a sliding trajectory of the limiting sliding block 221 can be limited, so that a rotation trajectory of two snake bone units can be limited, therefore ensuring smooth and stable rotation process; on the other hand, a maximum slip amount of the limiting sliding block 221 can be limited by an abutment between the limiting sliding block 221 and a side wall of the limiting sliding groove 211, therefore a maximum rotation angle of the snake bone unit is limited, so as to avoid a collision and an interference between two adjacent snake bone units caused by an excessive rotation angle of the snake bone tube.

In addition, the auxiliary sliding block 212 of one snake bone unit is capable of matching with and sliding relative to the auxiliary sliding groove 222 of another snake bone units. It can also play a same role as the limiting sliding block 221 and the limiting sliding groove 211 mentioned above, which can further increase a stability of the snake bone tube when it rotates.

Moreover, in the related art, when the snake bone unit rotates to a limit position, if the snake bone unit is further stressed, an external force directly acts on the limiting sliding block 221, and the limiting sliding block 221 is easily deformed due to the external force, and a strength of the connecting portion of the snake bone tube becomes lower.

In the present invention, by providing the auxiliary sliding block 212 and the corresponding auxiliary sliding groove 222, when the snake bone unit rotates to the limit position, the limiting sliding block 221 abuts against the side wall of the limiting sliding groove 211 and the auxiliary sliding block 212 abuts against the side wall of the auxiliary sliding groove 222. At this time, when the snake bone unit is further stressed, an external force is shared by the limiting sliding block 221 and the auxiliary sliding block 212. Therefore, the limiting sliding block 221 and the auxiliary sliding block 212 are not easily deformed, thus increasing the strength of the connecting portion of the snake bone tube.

It can be understood that by increasing the strength of the connecting portion of the snake bone tube, an original overall strength of the snake bone tube can be maintained by the snake bone unit with a less thickness. And the less a thickness of the snake bone unit is, the greater a cross-sectional area of the snake bone unit is, and the greater an available space of the instrument channel in the snake bone tube for accommodating instrument is, so as to better meet a requirement of liquid injection or instrument access. In addition, when a plurality of the auxiliary sliding block 212 is provided, the auxiliary sliding grooves 222 are provided with corresponding numbers, and the auxiliary sliding grooves 222 are not in communication with each other, to ensure that when the two snake bone units rotate to the limit position, each of the auxiliary sliding block 212 can abut against the corresponding side wall of the auxiliary sliding groove 222 to share the external force.

All of the rotating groove 22, the limiting sliding groove 211 and the auxiliary sliding groove 222 penetrates through a sidewall of the annular tube 100 along a radial direction of the annular tube 100. The snake bone unit is formed by laser cutting. By providing the rotating groove 22, the limiting sliding groove 211 and the auxiliary sliding groove 222 penetrating thought the sidewall of the annular tube 100 in the radial direction of the annular tube 100, a difficulty of laser cutting can be effectively reduced, so as to facilitate a processing and a molding of the snake bone tube.

In an embodiment, referring to FIG. 1, the auxiliary sliding block 212 is located on a side of the rotating block 21 away from the rotating groove 22. The auxiliary sliding groove 222 is located between the rotating block 21 and the rotating groove 22. Because a relatively great space for providing the auxiliary sliding groove 222 is provided between the rotating block 21 and the rotating groove 22, a size of the auxiliary sliding groove 222 can be increased as much as possible at the space between the rotating block 21 and the rotating groove 22. Thus, a volume of the auxiliary sliding block 212 capable of matching with and sliding relative to the auxiliary sliding groove 222 can be increased accordingly, and an ultimate pressure that the auxiliary sliding block 212 can bear can be increased, and the strength of the connecting portion can be further improved.

Referring to FIG. 1, both the limiting sliding block 221 and the auxiliary sliding block 212 are fixedly provided with a mousing hook 23. The limiting sliding groove 211 and the auxiliary sliding groove 222 are both connected to and in communication with an anti-dropping groove 24 corresponding to the mousing hook 23. When the snake bone unit rotates to the limit position, the limiting sliding block 221 rotates to a position abuts against the side wall of the limiting sliding groove 211. The mousing hook 23 is inserted into the anti-dropping groove 24. At this time, the abutment between the mousing hook 23 and a side wall of the anti-dropping groove 24 can effectively prevent a snake bone unit separating from another snake bone unit along an axis of the snake bone tube, thus increasing a connection stability of the two adjacent snake bone units.

Referring to FIG. 1, in the present embodiment, the limiting sliding grooves 211 and the auxiliary sliding grooves 222 are arc-shaped, and a center of the arc is a center of the rotating block 21. With this arrangement, when the rotating block 21 rotates in the rotating groove 22, rotation paths of the limiting sliding block 221 of the snake bone unit and the auxiliary sliding block 212 of the snake bone unit coincide with the corresponding limiting sliding groove 211 of another snake bone unit and the auxiliary sliding groove 222 of another snake bone unit. Therefore, the limiting sliding block 221 and the auxiliary sliding block 212 can slide smoothly to ensure smooth rotation between the two snake bone units.

Referring to FIG. 1 again, arc angles of the two limiting sliding grooves 211 are the same with the arc angle of the auxiliary sliding grooves 222, so as to ensure that when the snake bone unit is centered on the center of the rotating block 21 and rotated along any direction until one of the limiting sliding blocks 221 abuts against the side wall of one of the limiting sliding groove 211, the other limiting sliding blocks 221 can abut right against the side wall of the other limiting sliding grooves 211, and the auxiliary sliding block 212 can abut against the side wall of the auxiliary sliding groove 222.

Thus, when the snake bone unit rotates to the limit position in any direction, the auxiliary sliding block 212 and the two limiting sliding blocks 221 can share the external force, thus increasing strength and stability of the connecting portion of the snake bone unit.

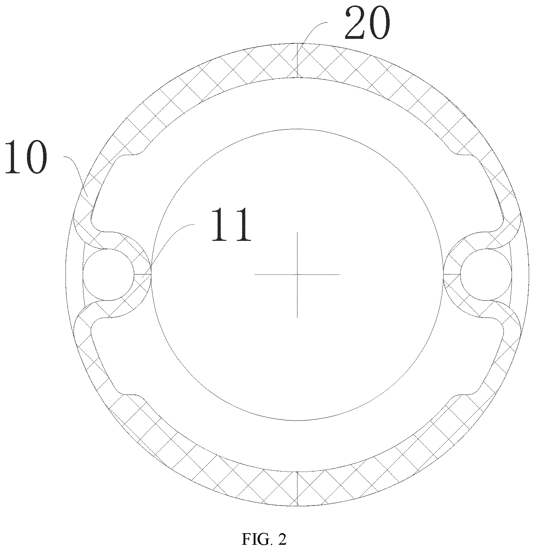

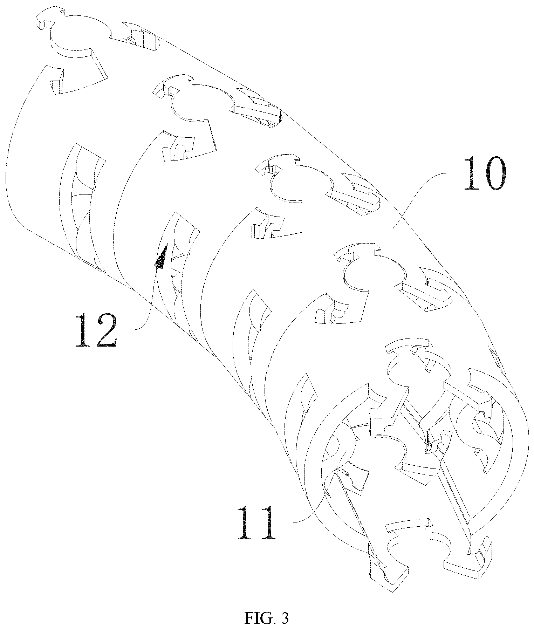

Referring to FIG. 2 and FIG. 3, the annular tube 100 includes a thin segment 10 and a thick segment 20 along a circumference of the annular tube 100. And a thickness of the thin segment 10 is less than a thickness of a thick segment 20. Both the rotating block 21 and the rotating groove 22 are disposed in the thick segment 20. The thin segment 10 includes a rope groove portion 11 (the thickness differences between the thick segment 20 and the thin segment 10 are not shown in FIG. 3).

By thinning the thickness of the thin segment 10, an internal space in the snake bone unit can be increased, so that an external diameter of the snake bone unit in the present invention will be less than an external diameter of the snake bone unit in the related art with the same internal space, thus achieving an effect of reducing the external diameter of a bending portion of the endoscope, which is beneficial to operation.

In addition, the rotating block 21 and the rotating groove 22 are arranged on the thick segment 20, that is, the connecting portion is arranged on the thick segment 20. Thus, only reducing the thickness of the thin segment 10 will not affect the strength of the connecting portion bearing the stressed. Therefore, the strength of the snake bone unit will not be greatly affected while increasing an internal space of the snake bone.

Referring to FIG. 1 to FIG. 2, the thin segment 10 is provided with two arc grooves 12 penetrating through the thin segment 10 along a radial direction of the annular tube 100, and the two arc grooves 12 are parallel to each other and arranged along a circumference of the snake bone unit. A part of the annular tube 100 located between each of the two arc grooves 12 bends inwards to an axis of the annular tube 100 to define the rope groove portion 11.

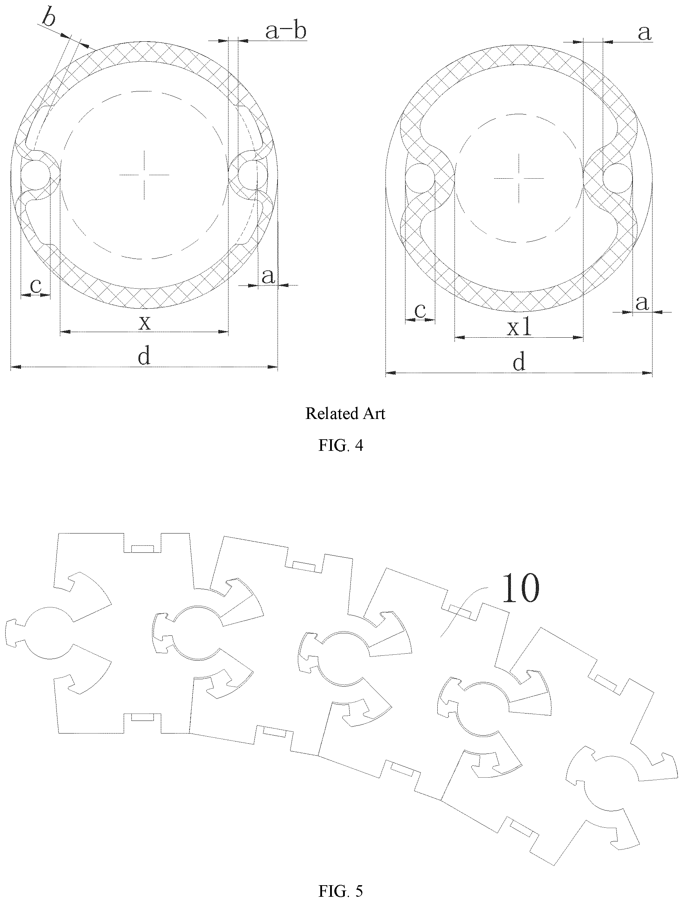

Referring to FIG. 4, the snake bone unit in the present invention is compared with the snake bone unit in the related art. Hypothetically, an external diameter of the snake bone tube in the present invention is the same as an external diameter of the snake bone tube in the related art, and defined as d; and a diameter of the groove through which a steel wire rope penetrates through of the snake bone tube in the present invention and the diameter of the groove through which a steel wire rope penetrates through of the snake bone tube in the related art are the same, and defined as c; a thickness of the annular tube 100 in the related art and a thickness of the thick segment 20 in the present invention are the same, and defined as $a$. In the present invention, the side wall of the thin segment 10 of the snake bone unit is thinned, and a thinned amount is defined as b. Therefore, the wall thickness of the thin segment 10 in the present invention is defined as $a\text{-}b$. A smallest diameter of the circular space of the snake bone unit in the present invention is defined as $x$, and a smallest diameter of the circular space of the snake bone unit in the related art is defined as $x_1$; Then, the diameter of the smallest circular space of the snake bone unit in the present invention $x$ satisfies a following formula:

$$x = \left[\frac{d}{2} - (a - b) - c - (a - b)\right] \times 2,$$

the diameter of the smallest circular space of the snake bone unit in the related art $x_1$ satisfies a following formula:

$$x_1 = \left[\frac{d}{2} - a - c - a\right] \times 2,$$

thus, the smallest diameter of the circular space of the snake bone unit in the present invention $x$ and the smallest diameter of the circular space of the snake bone unit in the related art $x_1$ satisfy a following formula: $x - x_1 = 4b$.

That is, in the present invention, by thinning the side wall of the thin segment 10, and the thinned amount is defined as $b$, the diameter of the smallest circular space in the snake bone tube can be increased by $4b$, thus greatly increasing the internal space of the snake bone tube.

In an embodiment, referring to FIG. 2, the number of the thick segment 20 is two, and two thick segments 20 are arranged symmetrically relative to an axis of the annular tube 100, one rotating block 21 is fixedly disposed at one end of the two thick segments 20, and a rotating groove 22 is disposed at the other end of the two thick segments 20. In this way, connection stability between two adjacent snake bone units is ensured by setting two groups of connecting portions symmetrically disposed relative to the axis of the annular tube 100.

In the above embodiment, the annular tube 100 includes two thin segments 10, the two thin segments 10 and the two thick segments 20 are disposed at intervals, and each of the thin segments 10 are provided with a rope groove portion 11.

The present invention further provides a snake bone tube. Referring to FIG. 5, the snake bone tube includes a plurality of snake bone units connected to and communicated with each other in sequence.

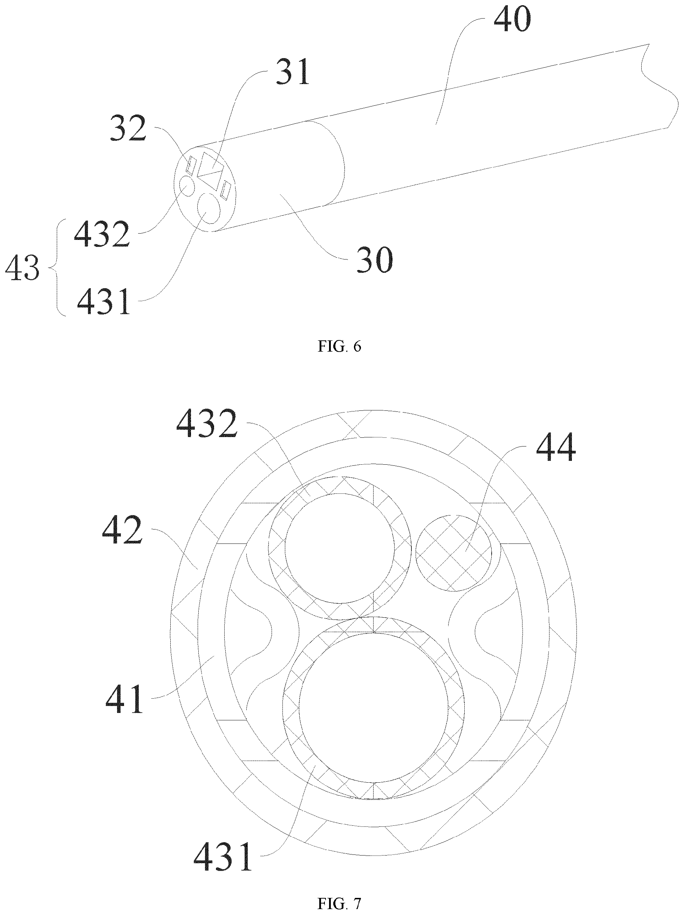

The present invention further provides a snake bone tube. Referring to FIG. 6 and FIG. 7, a cross section of the snake bone tube 41 is oval-shaped, and a conducting wire 44 and at least two channels 43 are arranged in the snake bone tube 41, and both the conducting wire 44 and the two channels 43 penetrate through the snake bone tube 41.

Referring to FIG. 7, the above embodiments can meet the requirement that the insertion catheter can be bent when in use. Because the bending part 40 includes the snake bone tube 41, after the bending portion 40 enters a natural channel of human body, the snake bending portion 40 can bend to a certain extent to fit the natural channel of the human body. The snake bone tube 41 facilitates the bending portion 40 to enter the natural channel of the human body, thus ensuring that a front end portion 30 can reach a surgical position.

In the present invention, by providing the conducting wire 44 and at least two channels 43 in the snake bone tube 41, a channel 43 for injecting liquid can be separated from a channel 43 for installing medical devices. Therefore, during operation, an injection of physiological saline and medical instruments will not interfere with each other, thus making the injection of physiological saline smoother. The injected physiological saline can fully dilute blood and drain tiny stones out of the body, so as to ensure that images taken by an image sensor 31 are clear and the tiny stones can be discharged in time, thus reducing a possibility of misjudgment by doctors and shortening operation time.

In addition, different channels 43 can also be loaded with different medical instruments, so as to realize a combination of different medical instruments and meet different surgical requirements. The different channels 43 can be infused with physiological saline according to actual needs after loading the corresponding medical instruments to meet the surgical requirements in different situations. For example, in related art, in the process of holmium laser lithotripsy, some stones float in renal pelvis. When a laser fiber is aimed at the stones and a laser pulse is turned on, the stones are easy to bounce off and float in the renal pelvis, which leads to a low efficiency of lithotripsy.

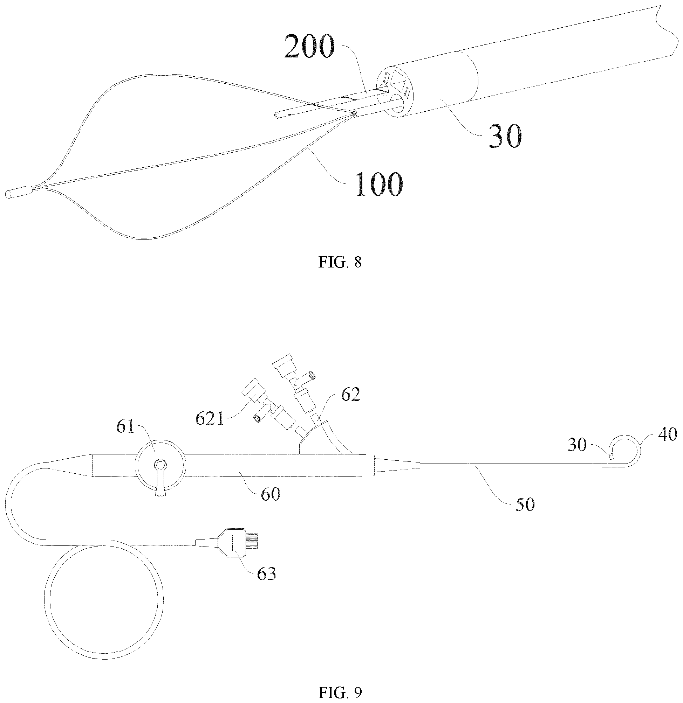

In some embodiments, referring to FIG. 8, a stone basket 300 and a holmium laser fiber 400 are installed in different channels 43, respectively, so that stones can be trapped by the stone basket 300 to prevent stones from floating during the operation. Then, the holmium laser fiber 400 is configured to turn on the laser pulse to perform lithotripsy, so as to avoid a reduction of operation efficiency caused by floating stones and an occurrence of laser accidental shooting to damage gaps of human organs, and achieve an effect of increasing operation efficiency and safety.

Referring to FIG. 7, in the related art, under conditions that one channel 43 is provided in the insertion catheter, a large gap is left between the channel 43 and the conducting wire 44. In the present invention, by providing at least two channels 43, one channel 43 can be similar in size to a single channel 43 in the related art, and the other channels 43 can be arranged in a gap between the channel 43 and the conducting wire 44, so as to utilize the gap space left under conditions one single channel 43 is provided.

With above arrangement, under a condition that an inner diameter of the snake bone tube 41 is unchanged, an overall cross-sectional area of the channel 43 is larger, and an utilization rate of a space in the snake bone tube 41 is greater, so that in the insertion catheter with the same size as that in the related art, at least two of the channel 43 can be arranged in the snake bone tube 41 in the present invention. Thus, the requirements of liquid injection and instrument access can be met in a limited space inside the snake bone tube 41.

For convenience of description, a space in the snake bone tube 41 between the conducting wire 44, the channel 43 and any two of the channels 43 are defined as gap space.

In the present invention, the conducting wires 44 and the channels 43 are filled in the snake bone tube 41, so that a channel 43 having a minimum size cannot be put into the gap space, therefore ensuring that the space inside the snake bone tube 41 is fully utilized. The minimum size here refers to the channel with an inner diameter that can meet the minimum requirements for injecting liquid or introducing medical devices.

In addition, compared with the circular-shaped snake bone tube 41 with the same area, by providing the snake bone tube 41 with an oval-shaped cross section, when a plurality of the channel 43 is arranged in the snake bone tube 41, an inner surface of the snake bone tube 41 is more adjacent to the channel 43 and the conducting wire 44. That is, the gap space is less, a space utilization rate in the snake bone tube 41 can be further increased.

In some embodiments, a cross-sectional shape of the channel 43 can be circular-shaped, oval-shaped or irregular-shaped. The specific cross-sectional shape of the channel 43 can be comprehensively determined according to an internal diameter of the snake bone tube 41, the number of the channels 43 and the arrangement structure between the channels 43 and the conducting wires 44. Generally, it is optionally to choose a shape that a minimum gap space is formed after the channels 43 are arranged into the snake bone tube 41, so as to make full use of the space in the snake bone tube 41.

In some embodiments, a perimeter of the cross section of the channel is in a range of 1Fr to 9Fr, so as to avoid that the size of the cross section of the channel 43 is too small, resulting in that the channel 43 cannot meet the minimum requirements for injecting liquid and introducing instruments.

"Size" in the present invention refers to a length of a longest line segment that can be made after two points on the cross section are arbitrarily determined and connected.

In an embodiment, Referring to FIG. 7, the channel 43 includes a first channel 431 and a second channel 432. And a cross-sectional area of the first channel 431 is larger than or equal to a cross-sectional area of the second channel 432. In an embodiment, the cross-sectional area of the first channel 431 is larger than the cross-sectional area of the second channel 432.

Because cross-sectional area requirements of the channel 43 are different when injecting liquid and loading medical instruments with different sizes, the channel 43 can include the first channel 431 and the second channel 432 with different cross-sectional areas, which can meet different requirements, that is, the requirements of injecting liquid and loading medical instruments with different sizes can be met at the same time, thus achieving an effect of increasing versatility.

For example, the first channel 431 is configured to loading medical instruments, while the second channel 432 is configured to injecting physiological saline. Optionally, the first channel 431 is configured to loading medical devices requiring a large space such as the stone basket 300, and the second channel 432 is configured to loading medical devices requiring a small space such as the holmium laser fiber 400.

The number of the first channels 431 and the second channels 432 in the snake bone tube 41 can be comprehensively determined according to the inner diameter of the snake bone tube 41, an external diameter of the conducting wire 44 and the arrangement structure between the channel 43 and the conducting wire 44, as long as the conducting wire 44 and the channel 43 can fill the snake bone tube 41 and make it impossible to put a channel 43 having a minimum size in the gap space.

In an embodiment, the snake bone tube 41 includes one first channel 431 and one to three the second channels 432.

The present invention further provides an insertion catheter, the insertion catheter includes a bending portion 40 and a front end portion 30. The front end portion 30 is provided with an image sensor 31 and a luminous source 32. The bending portion includes a flexible tube and the above snake bone tubes, the snake bone tube is wrapped in the flexible tube, the channel penetrates through the front end portion, and the conducting wire is electrically connected with the image sensor and the luminous source.

Referring to FIG. 6 and FIG. 7, the conducting wire 44 is electrically connected with the image sensor 31 and the luminous source 32, that is, power can be supplied to the image sensor 31 and the luminous source 32 through the conducting wire 44. In addition, an image signal captured by the image sensor 31 can be transmitted through the conducting wire 44 to meet visual requirements of the insertion catheter. After the front end portion 30 reaches a surgical position, the luminous source 32 emits light to provide brightness, with the image sensor 31 shooting pictures, an image signal of the surgical position can be transmitted to an external display device, so that it is convenient for doctors to observe and operate.

The flexible tube 42 is wrapped around the snake bone tube 41, which means that the flexible tube 42 is tightly attached to an outer surface of the snake bone tube 41, and the flexible tube 42 has a uniform thickness, so that an outer surface of the flexible tube 42 is also oval-shaped. Thus, at the same time the flexible tube 42 wraps the snake bone tube 41, the cross-sectional area of the flexible tube 42 is reduced as much as possible.

In some embodiments, the flexible tube 42 wrapped around the snake bone tube 41 can effectively prevent the snake bone tube 41 from scratching or pinching the inner wall of the natural channel when it is inserted into a natural channel of the human body. The cross-sectional area of the flexible tube 42 is as small as possible, which can facilitate the flexible tube 42 to enter the natural channel of the human body.

In some embodiments, both a perimeter of a cross section of the bending portion 40 and a perimeter of a cross section of the front end portion 30 are in a range of 6Fr to 18Fr, so as to avoid a situation that the bending portion 40 and the front end portion 30 cannot enter the natural channel of the human body due to their too large cross-sectional areas; and avoid a situation that an internal space of the snake bone tube 41 is not enough to set the channel 43 and the conducting wire 44 due to too small cross-sectional areas of the bending portion 40 and the front end portion 30.

In an embodiment, the perimeter of the cross section of the bending portion 40 is 9Fr and the perimeter of the cross section of the front end portion 30 is 9.3Fr.

The present invention further provides an electronic endoscope, referring to FIG. 9, the electronic endoscope includes an operating handle 60, an inserting portion 50 and the above insertion catheters. One end of the inserting portion 50 is connected with the bending portion 40, and the other end is connected with the operating handle 60, and the operating handle 60 is provided with a toggling handle 61 for controlling a bending of the bending portion 40.

The toggling handle 61 controls the bending of the bending portion 40 by controlling the snake bone tube 41 to bend in up direction or down direction in FIG. 9, so that the bending portion 40 can fit the natural channel of human body, achieving an effect of facilitating the bending portion 40 to enter the natural channel of human body and ensuring that the front end portion 30 can reach the surgical position.

A perimeter of a cross section of the inserting portion 50 is also in a range of 6Fr to 18Fr, so as to avoid conditions that the inserting portion cannot enter the natural channel of human body due to a too large a cross section area of the inserting portion 50, and avoid conditions that the internal space is insufficient to dispose the channel 43 and the conducting wire 44 due to too small cross-sectional area of the inserting portion 50. In one embodiment, the perimeter of the cross section of the inserting portion 50 is 8.4Fr.

In some embodiments, the operating handle 60 is provided with the same number of insetting ports 62 as the channels 43, and each of the channels 43 penetrates through the inserting portion 50 and the operating handle 60 and is in communication with one of the insetting ports 62. The insetting ports 62 are configured to connect corresponding connectors 641. The connector 641 includes an instrument connector and a liquid injection connector according to a function of the channel 43 connected to the setting port 62, so as to meet an insertion requirement of medical instruments and injection requirements of liquids such as physiological saline.

In some embodiments, the operating handle 60 is further provided with a plug 63, and the conducting wire 44 penetrates through the inserting portion 50 and the operating handle 60, is electrically connected with the plug 63. The plug 63 can be electrically connected with a dedicated controller and display, so that an image signal captured by the image sensor 31 can be transmitted to a corresponding display through the conducting wire 44 and the plug 63, thus realizing a visual function.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present invention.

One of ordinary skill in the art should recognize that the above embodiments are used only to illustrate the present invention and are not used to limit the present invention, and that appropriate variations and improvements to the above embodiments fall within the protection scope of the present invention so long as they are made without departing from the substantial spirit of the present invention.

**Claims**

1. A snake bone unit, **characterized by** comprising an annular tube, wherein one end of the annular tube is fixed with a rotating block, the other end of the annular tube is provided with a rotating groove, and the rotating block is capable of being connected with a rotating groove of other snake bone units and rotating relative to the rotating groove of other snake bone units;

   two sides of the rotating groove are fixedly provided with a limiting sliding block, respectively, two sides of the rotating block are provided with a limiting sliding groove, respectively, and the limiting sliding block is capable of being connected to a limiting sliding groove of the other snake bone units and sliding relative to the limiting sliding groove of the other snake bone units; the rotating block is fixedly provided with at least one auxiliary sliding block, the annular tube is correspondingly provided with auxiliary sliding grooves with a same number as the at least one auxiliary sliding block, the auxiliary sliding grooves are in communication with the rotating groove, and each of the at least one auxiliary sliding block is capable of matching with an auxiliary sliding groove of the other snake bone units and sliding relative to the auxiliary sliding groove of the other snake bone units.

2. The snake bone unit of claim 1, wherein each of the at least one auxiliary sliding block is located at a side of the rotating block away from the rotating groove, and each of the auxiliary sliding grooves is located between the rotating block and the rotating groove.

3. The snake bone unit of claim 1, wherein both the limiting sliding block and the at least one auxiliary sliding block are fixedly provided with a mousing hook, respectively, and both the limiting sliding groove and the auxiliary sliding grooves are provided with an anti-dropping groove corresponding to the mousing hook respectively, and the anti-dropping groove is in communication with the limiting sliding groove and the auxiliary sliding grooves, respectively.

4. The snake bone unit of claim 1, wherein the number of the limiting sliding groove is two, both the two limiting sliding grooves and the auxiliary sliding grooves are circular arc-shaped, an arc center of the two limiting sliding grooves is a center of the rotating block, and an arc center of the auxiliary sliding groove is a center of the rotating groove.

5. The snake bone unit of claim 4, wherein arc angles of the two limiting sliding grooves are the same with arc angles of the auxiliary sliding grooves.

6. The snake bone unit of claim 1, wherein the annular tube comprises a thin segment and a thick segment along a circumference of the annular tube, and a thickness of the thin segment is less than a thickness of the thick segment, both the rotating block and the rotating groove are located in the thick segment, and the thin segment is provided a rope groove portion.

7. The snake bone unit of claim 6, wherein the thin segment is provided with two arc grooves which are parallel to each other and arranged along the circumference of the annular tube, the two arc grooves penetrates through a side wall of the thin segment, and the rope groove portion is formed by a part of the annular tube located between each of the two arc grooves bending inwards to an axis of the annular tube.

8. The snake bone unit of claim 6, wherein the annular tube comprises two thick segments, and the two thick segments are arranged symmetrically relative to an axis of the annular tube, one rotating block is fixedly disposed at an end of each of the two thick segments, and one rotating groove is disposed at the other end of each of the two thick segments.

9. The snake bone unit of claim 8, wherein the annular tube comprises two thin segments, the two thin segments and the two thick segments are disposed at intervals, and each of the two thin segments is provided with a rope groove portion.

10. A snake bone tube, **characterizing by** a cross section of the snake bone tube is oval-shaped, and a conducting wire and at least two channels are arranged in the snake bone tube, and the conducting wire and the at least two channels penetrate through the snake bone tube along an axis of the snake bone tube.

11. The snake bone tube of claim 10, wherein the snake bone tube comprises a plurality of snake bone units connected to and in communication with each other in sequence, each of the plurality of snake bone units comprises an annular tube,

one end of the annular tube is fixed to a rotating block, the other end of the annular tube is provided with a rotating groove, and the rotating block is capable of being connected with a rotating groove of other snake bone units and rotating relative to the rotating groove of other snake bone units;
two sides of the rotating groove are fixedly provided with a limiting sliding block, respectively, two sides of the rotating block are provided with a limiting sliding groove, respectively, and the limiting sliding block is capable of being connected to a limiting sliding groove of the other snake bone units and sliding relative to the limiting sliding groove of the other snake bone units;
the rotating block is fixedly provided with at least one auxiliary sliding block, the annular tube is correspondingly provided with auxiliary sliding grooves with a same number as the at least one auxiliary sliding block, the auxiliary sliding grooves are in communication with the rotating groove, and the at least one auxiliary sliding blocks are capable of matching with an auxiliary sliding groove of the other snake bone units and sliding relative to the auxiliary sliding groove of the other snake bone units.

12. The snake bone tube of claim 11, wherein each of the at least one auxiliary sliding block is located at a side of the rotating block away from the rotating groove, and each of the auxiliary sliding grooves is located between the rotating block and the rotating groove.

13. The snake bone tube of claim 11, wherein both the limiting sliding block and the at least one auxiliary sliding block are fixedly provided with a mousing hook, respectively, and both the limiting sliding groove and the auxiliary sliding grooves are provided with an anti-dropping groove corresponding to the mousing hook respectively, and the anti-dropping groove is in communication with the limiting sliding groove and the auxiliary sliding grooves, respectively.

14. The snake bone tube of claim 11, wherein the number of the limiting sliding groove is two, both the two limiting sliding grooves and the auxiliary sliding grooves are circular arc-shaped, an arc center of the two limiting sliding grooves is a center of the rotating block, and an arc center of the auxiliary sliding groove is a center of the rotating groove.

15. The snake bone tube of claim 14, wherein arc angles of the two limiting sliding grooves are the same with arc angles of the auxiliary sliding grooves.

16. The snake bone tube of claim 11, wherein the annular tube comprises a thin segment and a thick segment along a circumference of the annular tube, and a thickness of the thin segment is less than a thickness of the thick segment, both the rotating block and the rotating groove are located in the thick segment, and the thin segment is provided a rope groove portion.

17. The snake bone tube of claim 16, wherein the thin segment is provided with two arc grooves which are parallel to each other and arranged along the circumference of the annular tube, and the rope groove portion is defined by a part of the annular tube located between each of the two arc grooves bending inwards to an axis of the annular tube.

18. The snake bone tube of claim 16, wherein the annular tube comprises two thick segments, and the two thick segments are arranged symmetrically relative to an axis of the annular tube, one rotating block is fixedly disposed at an end of each of the two thick segments, and one rotating groove is disposed at the other end of each of the two thick segments.

19. The snake bone tube of claim 18, wherein the annular tube comprises two thin segments, the two thin segments and the two thick segments are disposed at intervals, and each of the two thin segments is provided with a rope groove portion.

20. The snake bone tube of claim 10, wherein a shape of a cross section of at least two channels is circular-shaped, oval-shaped or irregular-shaped.

21. The snake bone tube of claim 10, wherein a perimeter of the cross section of at least two channels is in a range of 1Fr to 9Fr.

22. The snake bone tube of claim 10, wherein the at least two channels comprises a first channel and a second channel.

23. The snake bone tube of claim 22, wherein a cross-sectional area of the first channel is greater than or equal to a cross-sectional area of the second channel.

24. The snake bone tube of claim 22, wherein the number of the first channel is one, and the number of the second channel is in a range of one to three.

25. An insertion catheter, **characterizing by** comprising a bending portion and a front end portion, the front end portion is provided with an image sensor and a luminous source;
the bending portion comprises a flexible tube and the snake bone tube of any one of claims 10 to 24, the snake bone tube is wrapped in the flexible tube, the at least two channels penetrate through the front end portion, and the conducting wire is electrically connected with the image sensor and the luminous source.

26. The insertion catheter of claim 25, wherein both a perimeter of a cross section of the bending portion and a perimeter of a cross section of the front end portion are in a range of 6Fr to 18Fr.

27. An electronic endoscope, **characterizing by** comprising an operating handle, an inserting portion and the insertion catheter of claims 25 or 26, one end of the inserting portion is connected with the bending portion, and the other end of the inserting portion is connected with the operating handle, and the operating handle is provided with a toggling handle configured for controlling the bending portion to bend.

28. The electronic endoscope of claim 27, wherein the operating handle is provided with an inserting port, the number of the inserting port is the same as the number of the at least two channels, and each of the at least two channels penetrates through the inserting portion and the operating handle until to be in communication with the inserting port.

29. The electronic endoscope of claim 27, wherein the operating handle is further provided with a plug, and the conducting wire penetrates through the inserting portion and the operating handle, and is electrically connected with the plug.

FIG. 1

FIG. 2

FIG. 3

Related Art

FIG. 4

FIG. 5

FIG. 6

FIG. 7

FIG. 8

FIG. 9

| | | |
|---|---|---|
| **INTERNATIONAL SEARCH REPORT** | International application No. | |
| | **PCT/CN2022/113677** | |

**A.     CLASSIFICATION OF SUBJECT MATTER**

A61B 1/005(2006.01)i

According to International Patent Classification (IPC) or to both national classification and IPC

**B.     FIELDS SEARCHED**

Minimum documentation searched (classification system followed by classification symbols)

A61B1

Documentation searched other than minimum documentation to the extent that such documents are included in the fields searched

Electronic data base consulted during the international search (name of data base and, where practicable, search terms used)

CNABS, CNTXT: 蛇骨, 弯曲, 槽, 凹, 通道, 管, 腔, 椭圆, 导线, 线缆, 电缆, 电线; VEN, USTXT, WOTXT, EPTXT: bend, block, groove, concave, passageway, tube, cavity, oval, ellipse, wire

**C.     DOCUMENTS CONSIDERED TO BE RELEVANT**

| Category* | Citation of document, with indication, where appropriate, of the relevant passages | Relevant to claim No. |
|---|---|---|
| PX | CN 216933131 U (ZHEJIANG HEALNOC TECHNOLOGY CO., LTD.) 12 July 2022 (2022-07-12) <br> claims 1-10 | 1-9 |
| PX | CN 217066329 U (WEICHUANG YOUTONG MEDICAL TECHNOLOGY (SHANGHAI) CO., LTD.) 29 July 2022 (2022-07-29) <br> description, paragraphs 31-63, and figures 1-4 | 1-9 |
| PX | CN 114847845 A (ZHEJIANG BENSHU TECHNOLOGY CO., LTD.) 05 August 2022 (2022-08-05) <br> description, paragraphs 43-74, and figures 1-9 | 10, 20-29 |
| PY | CN 114847845 A (ZHEJIANG BENSHU TECHNOLOGY CO., LTD.) 05 August 2022 (2022-08-05) <br> description, paragraphs 43-74, and figures 1-9 | 11-19 |
| PY | CN 216933131 U (ZHEJIANG HEALNOC TECHNOLOGY CO., LTD.) 12 July 2022 (2022-07-12) <br> claims 1-10 | 11-19 |

☑ Further documents are listed in the continuation of Box C.     ☑ See patent family annex.

| | |
|---|---|
| *     Special categories of cited documents: <br> "A"     document defining the general state of the art which is not considered to be of particular relevance <br> "E"     earlier application or patent but published on or after the international filing date <br> "L"     document which may throw doubts on priority claim(s) or which is cited to establish the publication date of another citation or other special reason (as specified) <br> "O"     document referring to an oral disclosure, use, exhibition or other means <br> "P"     document published prior to the international filing date but later than the priority date claimed | "T"     later document published after the international filing date or priority date and not in conflict with the application but cited to understand the principle or theory underlying the invention <br> "X"     document of particular relevance; the claimed invention cannot be considered novel or cannot be considered to involve an inventive step when the document is taken alone <br> "Y"     document of particular relevance; the claimed invention cannot be considered to involve an inventive step when the document is combined with one or more other such documents, such combination being obvious to a person skilled in the art <br> "&"     document member of the same patent family |

| Date of the actual completion of the international search | Date of mailing of the international search report |
|---|---|
| **31 October 2022** | **14 November 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.

**PCT/CN2022/113677**

**C. DOCUMENTS CONSIDERED TO BE RELEVANT**

| Category* | Citation of document, with indication, where appropriate, of the relevant passages | Relevant to claim No. |
|---|---|---|
| PY | CN 217066329 U (WEICHUANG YOUTONG MEDICAL TECHNOLOGY (SHANGHAI) CO., LTD.) 29 July 2022 (2022-07-29) description, paragraphs 31-63, and figures 1-4 | 11-19 |
| X | WO 2020135362 A1 (SHENZHEN XIANZAN TECHNOLOGY CO., LTD.) 02 July 2020 (2020-07-02) description, pages 4-9, and figures 1-11 | 1-9 |
| X | US 2016022960 A1 (TERUMO KABUSHIKI KAISHA) 28 January 2016 (2016-01-28) description, paragraphs 50-77, and figures 1-6C | 1-9 |
| X | CN 108078535 A (ZHENGZHOU RUNDE PHOTOELECTRIC TECHNOLOGY CO., LTD.) 29 May 2018 (2018-05-29) description, paragraphs 44-73, and figures 1-6 | 1-9 |
| X | CN 105852781 A (FUJIFILM CORP.) 17 August 2016 (2016-08-17) description, paragraphs 60-75, and figures 1-9 | 10, 20-29 |
| X | CN 108742476 A (ZHUHAI MINDSION MEDICAL TECHNOLOGY CO., LTD.) 06 November 2018 (2018-11-06) description, paragraphs 19-26, and figures 1-4 | 10, 20-29 |
| Y | CN 105852781 A (FUJIFILM CORP.) 17 August 2016 (2016-08-17) description, paragraphs 60-75, and figures 1-9 | 11-19 |
| Y | CN 108742476 A (ZHUHAI MINDSION MEDICAL TECHNOLOGY CO., LTD.) 06 November 2018 (2018-11-06) description, paragraphs 19-26, and figures 1-4 | 11-19 |
| Y | WO 2020135362 A1 (SHENZHEN XIANZAN TECHNOLOGY CO., LTD.) 02 July 2020 (2020-07-02) description, pages 4-9, and figures 1-11 | 11-19 |
| Y | US 2016022960 A1 (TERUMO KABUSHIKI KAISHA) 28 January 2016 (2016-01-28) description, paragraphs 50-77, and figures 1-6C | 11-19 |
| Y | CN 108078535 A (ZHENGZHOU RUNDE PHOTOELECTRIC TECHNOLOGY CO., LTD.) 29 May 2018 (2018-05-29) description, paragraphs 44-73, and figures 1-6 | 11-19 |
| A | US 6364828 B1 (YEUNG, Hubert k.) 02 April 2002 (2002-04-02) entire document | 1-29 |
| A | CN 107091261 A (PUSEN MEDICAL TECHNOLOGY (ZHUHAI) CO., LTD.) 25 August 2017 (2017-08-25) entire document | 1-29 |

Form PCT/ISA/210 (second sheet) (January 2015)

INTERNATIONAL SEARCH REPORT
Information on patent family members

International application No.

**PCT/CN2022/113677**

| Patent document cited in search report | | | Publication date (day/month/year) | Patent family member(s) | | | Publication date (day/month/year) |
|---|---|---|---|---|---|---|---|
| CN | 216933131 | U | 12 July 2022 | None | | | |
| CN | 217066329 | U | 29 July 2022 | None | | | |
| CN | 114847845 | A | 05 August 2022 | None | | | |
| WO | 2020135362 | A1 | 02 July 2020 | CN | 109497915 | A | 22 March 2019 |
| | | | | CN | 209770314 | U | 13 December 2019 |
| US | 2016022960 | A1 | 28 January 2016 | JP | WO2014162608 | A1 | 16 February 2017 |
| | | | | WO | 2014162608 | A1 | 09 October 2014 |
| | | | | US | 9775967 | B2 | 03 October 2017 |
| CN | 108078535 | A | 29 May 2018 | CN | 209220212 | U | 09 August 2019 |
| CN | 105852781 | A | 17 August 2016 | US | 2016227985 | A1 | 11 August 2016 |
| | | | | JP | 2016144574 | A | 12 August 2016 |
| | | | | DE | 102016102156 | A1 | 11 August 2016 |
| | | | | US | 10076236 | B2 | 18 September 2018 |
| | | | | JP | 6423278 | B2 | 14 November 2018 |
| | | | | CN | 105852781 | B | 07 April 2020 |
| CN | 108742476 | A | 06 November 2018 | CN | 209059124 | U | 05 July 2019 |
| US | 6364828 | B1 | 02 April 2002 | None | | | |
| CN | 107091261 | A | 25 August 2017 | US | 2018231162 | A1 | 16 August 2018 |
| | | | | EP | 3360490 | A1 | 15 August 2018 |
| | | | | CN | 107091261 | B | 09 October 2020 |
| | | | | US | 10816118 | B2 | 27 October 2020 |
| | | | | EP | 3360490 | B1 | 01 July 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111308813

- CN 202122726820